(19) **Europäisches Patentamt / European Patent Office / Office européen des brevets**

(11) **EP 1 097 724 B1**

(12) **FASCICULE DE BREVET EUROPEEN**

(45) Date de publication et mention
de la délivrance du brevet:
**28.11.2007 Bulletin 2007/48**

(51) Int Cl.:
*A61M 1/34* (2006.01)

(21) Numéro de dépôt: **00420222.2**

(22) Date de dépôt: **27.10.2000**

(54) **Appareil d'hémofiltration**

Hämofiltrationsvorrichtung

Hemofiltration apparatus

(84) Etats contractants désignés:
**AT BE CH CY DE DK ES FI FR GB GR IE IT LI LU MC NL PT SE**

(30) Priorité: **08.11.1999 FR 9914253**

(43) Date de publication de la demande:
**09.05.2001 Bulletin 2001/19**

(73) Titulaire: **Gambro Industries SAS**
**69330 Meyzieu (FR)**

(72) Inventeur: **Bene, Bernard**
**69540 Irigny (FR)**

(74) Mandataire: **Sutto, Luca et al**
**Gambro Intellectual Property Department,**
**P.O. Box 98**
**41037 Mirandola (MO) (IT)**

(56) Documents cités:
**EP-A- 0 898 975          EP-A- 0 898 976**
**DE-A- 19 654 746**

**Description**

La présente invention a pour objet un appareil d'hémofiltration permettant de controler, de façon indépendante, la concentration d'au moins deux substances ioniques dans le milieu intérieur d'un patient

Les reins remplissent de multiples fonctions parmi lesquelles l'élimination d'eau, l'excrétion des catabolites (ou déchets du métabolisme, tels que l'urée, la créatinine), la régulation de la concentration des substances ioniques du sang (sodium, potassium, magnésium, calcium, bicarbonates, phosphates, chlorures) et la régulation de l'équilibré acido-basique du milieu intérieur qui est obtenue notamment par l'élimination des acides faibles (phosphates, acides monosodiques) et par la production de sels d'ammonium.

Chez les personnes qui ont perdu l'usage de leurs reins, ces mécanismes excréteurs et régulateurs ne jouant plus, le milieu intérieur se charge en eau et en déchets du métabolisme et présente un excès de certaines substances ioniques (de sodium en particulier), ainsi que, en général, une acidose, le pH du plasma sanguin se déplaçant vers 7 (le pH sanguin varie normalement dans des limites étroites comprises entre 7,35 et 7,45).

Pour pallier le dysfonctionnement des reins, on recourt classiquement soit à la dialyse, soit à l'hémofiltration, qui sont des traitements du sang administrés au moyen d'un échangeur à membrane semi-perméable (hémodialyseur/hémofiltre) qui est relié au patient par un circuit de circulation extracorporelle de sang.

La dialyse consiste à faire circuler, d'un côté et de l'autre de la membrane de l'échangeur, le sang du patient et un liquide de dialyse comprenant les principales substances ioniques du sang, dans des concentrations proches de celles du sang d'un sujet sain. Par ailleurs, un volume d'eau plasmatique déterminé correspondant au poids que le patient doit perdre lors de chaque séance de dialyse est induit à s'écouler par ultrafiltration au travers de la membrane dans le compartiment du liquide de dialyse. L'ultrafiltration résulte d'une différence de pression entretenue entre les deux compartiments de l'échangeur délimités par la membrane.

Le traitement du sang qui est effectué dans l'échangeur résulte du transfert diffusif, au travers de la membrane, des molécules d'une même substance (substances ioniques, déchets du métabolisme) ayant des concentrations différentes de part et d'autre de la membrane, les molécules migrant du liquide où leur concentration est la plus élevée vers le liquide où leur concentration est la moins élevée.

Dans un appareil de dialyse classique, le liquide de dialyse est préparé par un mélange dosé d'eau et de deux solutions concentrées, une première solution concentrée contenant du chlorure de sodium et du bicarbonate de sodium et la seconde solution concentrée contenant des chlorures de calcium, de potassium et de magnésium ainsi que de l'acide acétique. L'acide acétique a pour fonction de limiter la formation de précipités de carbonates de calcium et de magnésium qui forment des dépôts indésirables dans le circuit hydraulique de l'appareil de dialyse. D'autres dispositifs de dialyse ont été proposés, notamment dans les documents EP 0 898 975 et EP 0 898 976, où le liquide de dialyse utilisé ne contient pas de calcium ni de magnésium (respectivement de bicarbonate) et où une solution de bicarbonate de sodium (respectivement de chlorure de calcium et de magnésium) est perfusée au patient. Quel que soit le type de dialyse administré, au débit de liquide de dialyse usuel, soit 500 ml/min., ce sont cent vingt litres de liquide de dialyse qui sont préparés et utilisés au cours d'une séance de dialyse de quatre heures.

L'hémofiltration consiste à extraire du sang circulant dans l'échangeur, par ultrafiltration, un volume important (jusqu'à trente litres au cours d'une séance de quatre heures) d'eau plasmatique qui est remplacé partiellement au moyen d'une perfusion simultanée d'un liquide de substitution stérile.

Le traitement du sang qui est effectué dans l'échangeur résulte ici du transfert convectif des molécules (substances ioniques, déchets du métabolisme) qui sont entraînées par l'eau plasmatique qui filtre au travers de la membrane sous l'effet de la différence de pression créée entre les deux compartiments de l'échangeur.

La composition électrolytique du liquide de substitution utilisé en hémofiltration est identique à celle d'un liquide de dialyse. Ce liquide peut être fabriqué et conditionné par un laboratoire pharmaceutique sous la forme de deux récipients pour deux liquides stériles à mélanger juste avant utilisation, l'un des récipients contenant tout le bicarbonate et l'autre récipient contenant tout le calcium et le magnésium. Ce liquide de substitution peut aussi être préparé extemporanément par filtration d'un liquide de dialyse, comme cela est décrit notamment dans le document EP 0 622 087, dont l'objet est un appareil d'hémodiafiltration (combinaison des deux traitements définis plus haut): dans cet appareil, une partie du liquide de dialyse produit par un générateur de liquide de dialyse est injecté, après filtration, dans la canalisation de restitution du sang du circuit extracorporel, tandis que le reste du liquide de dialyse est mis en circulation dans l'échangeur.

Le document DE 196 54 746 décrit un appareil d'hémodiafiltration comprenant: des moyens pour préparer une première solution injectable à partir d'au moins une solution concentré; des moyens pour injecter, à un débit d'injection (exemple 40-50 ml/min), la première solution dans une canalisation de prélèvement de sang; des moyens pour perfuser à un patient une deuxième solution contenant au moins une substance ionique A (Ca++, Mg++, Cl-) ayant une concen-tration déterminée $[A]_{post}$ ($\neq 0$) différente de la concentration $[A]_{pre}$ (= 0) de cette substance A dans la première solution.

En dépit de son efficacité et de sa supériorité reconnue sur la dialyse au plan thérapeutique (voir notamment, Long-term morbidity: Hemofiltration vs. Hemodialysis, by E. Quellhorst, U. Hildebrand, A. Solf, in Contrib. Nephrol. Basel, Karger, 1995, vol 113, PP 110-119), l'hémofiltration souffre de deux limitations: une première limitation est liée au prix du traitement lorsque le liquide de substitution utilisé est acheté prêt à l'emploi. Comme ces solutions stériles conditionnées dans des récipients dédoublés sont chères, la tendance est à limiter le traitement administré à un échange de liquides n'excédant pas trente litres (soit quatre fois moins que le volume de liquide de traitement utilisé au cours d'une séance de dialyse). La seconde limitation, c'est que, même avec un appareil comme celui qui est décrit dans le docoment EP 622 087 mentionné plus haut, qui permet de disposer de liquide de substitution à moindre coût, le débit d'échange est limité puisque le débit d'ultrafiltration ne peut guère être fixé à plus d'un tiers du débit du sang dans le circuit extracorporel.

Au vu de ce qui précède, un but de l'invention est de réaliser un système de traitement de l'insuffisance rénale qui permette de réaliser une séance d'hémofiltration au cours de laquelle des volumes de liquide très importants puissent être échangés, sans que cette séance d'hémofiltration ne soit plus longue, ni plus onéreuse qu'une séance de dialyse classique, et sans qu'elle en présente non plus les inconvénients connus relatifs à la préparation d'un liquide de traitement instable.

Conformément à l'invention, ce but est atteint au moyen d'un appareil d'hémofiltration destiné à coopérer avec un hémofiltre ayant un premier et un second compartiments séparés par une membrane semi-perméable, le premier compartiment ayant une entrée connectable à une canalisation de prélèvement de sang et une sortie connectable à une canalisation de restitution de sang, et le second compartiment ayant une sortie connectable à un canalisation d'évacuation de liquide usé, cet appareil d'hémofiltration comportant:

-   des moyens pour préparer une première solution injectable à partir d'au moins une solution concentrée;
-   des moyens pour injecter, à un débit d'injection $Q_{pre}$, la première solution dans la canalisation de prélèvement de sang;
-   des moyens pour perfuser à un patient une deuxième solution contenant au moins une substance ionique A ayant une concentration déterminée $[A]_{pos}$t différente de la concentration $[A]_{pre}$ de cette substance A dans la première solution;
-   des moyens pour déterminer un débit de perfusion Qpost de la deuxième solution pour que la concentration de la substance A dans le milieu intérieur du patient tende vers une concentration souhaitée $[A]_{des}$, en fonction de la concentration $[A]_{post}$ de la substance A dans la deuxième solution, de la concentration souhaitée $[A]_{des}$, du débit d'injection $Q_{pre}$ de la première solution, de la concentration solution [A]pre de la substance A dans la première solution et du débit du sang QB.

L'invention permet d'atteindre le but poursuivi car, comme la première solution est injectée en amont de l'échangeur, le sang qui pénètre dans l'échangeur est dilué de sorte que la proportion de ce mélange qui peut être ultrafiltrée est très supérieure à la proportion admissible lorsque le sang n'est pas dilué. Par conséquent, on peut envisager avec cet appareil d'administrer un traitement au cours duquel cinquante à quatre-vingts litres de liquide de traitement sont échangés contre le même volume d'eau plasmatique. Par ailleurs, la dilution du sang réduit les inconvénients liés à la viscosité du sang total, à savoir la résistance à l'écoulement et l'entrave aux transferts, et elle diminue les risques de coagulation dans l'échangeur puisque la concentration des facteurs de coagulation s'y trouve réduite. L'ultrafiltration d'un sang dilué est aussi moins traumatique pour les éléments figurés du sang que l'ultrafiltration de sang non dilué.

Ce système présente en outre l'avantage que les concentrations respectives d'au moins deux substances ioniques entrant dans la composition d'un liquide de substitution peuvent être réglées indépendamment l'une de l'autre, ce qui est souvent souhaitable au moins pour le sodium, le potassium ou le bicarbonate.

Selon une caractéristique de l'invention, la substance A est absente de la première solution et les moyens de détermination du débit de perfusion Qpost comprennent des moyens de calcul pour calculer le débit de perfusion Qpost selon la formule :

$$Qpost = \frac{[A]_{des} \times Q_B \times Q_{pre}}{[A]post \times Q_B + [A]post \times Q_{pre} - [A]_{des} \times Q_B} \qquad (1)$$

Selon une autre caractéristique de l'invention, l'appareil d'hémofiltration comprend en outre des moyens de commande pour piloter les moyens de perfusion de façon que le débit de la solution de perfusion soit sensiblement égal au débit déterminé $Q_{post}$.

Selon un mode de réalisation de l'invention, l'une des première et deuxième solutions contient du bicarbonate et ne contient pas de magnésium ni de calcium, tandis que l'autre solution contient du magnésium et du calcium et ne contient pas de bicarbonate.

**EP 1 097 724 B1**

Grâce à la séparation du bicarbonate, d'une part, et du magnésium et du calcium d'autre part, il est inutile d'ajouter de l'acide acétique dans l'une ou l'autre des solutions pour éviter la formation de précipités indésirables, ce qui présente des avantages évidents en ce qui concerne les dispositifs utilisés pour préparer les solutions, qui ne sont exposés ni à la corrosion due à l'action d'un acide, ni à l'encrassement dû aux dépôts carbonatés. Par ailleurs, l'absence d'acide dans la première solution est souhaitable au plan thérapeutique puisqu'il semble que la dilution du sang avec un liquide ayant un pH inférieur à celui du sang soit l'un des cofacteurs de certaines réactions d'hypersensibilité.

Un autre intérêt de l'invention réside dans la possibilité de déterminer et de régler en continu le débit de la deuxième solution, en particulier lorsque cette solution contient tout le calcium, le magnésium et le potassium perfusé au patient. On sait en effet que l'excès comme le déficit de potassium, de calcium et de magnésium dans le sang peut engendrer chez le patient des troubles graves, notamment des troubles cardiaques. Or l'ultrafiltration du sang provoque l'élimination par convection d'une partie des substances ioniques du sang dilué qui circule dans l'échangeur et cette perte convective varie en fonction des paramètres du traitement qui sont modifiés en cours de traitement, tels que le débit de la première solution et le débit du sang. Grâce aux moyens de détermination du débit de la deuxième- solution dont est doté l'appareil d'hémofiltration selon l'invention il est possible de compenser en continu les pertes convectives variables qui se produisent dans l'échangeur.

La perfusion, en aval de l'échangeur ou directement dans le patient, d'une deuxième solution contenant tout le calcium perfusé au patient, présente encore un autre avantage résultant de l'appauvrissement en calcium du sang dilué dans l'échangeur: on sait que le calcium ionique intervient dans la cascade de réactions constituant le processus de coagulation du sang. L'appauvrissement massif du sang en calcium dans l'échangeur inhibe donc partiellement le processus de coagulation, ce qui permet de réduire la quantité d'anticoagulant injectée dans le circuit de circulation extracorporelle de sang pour y prévenir la coagulation du sang.

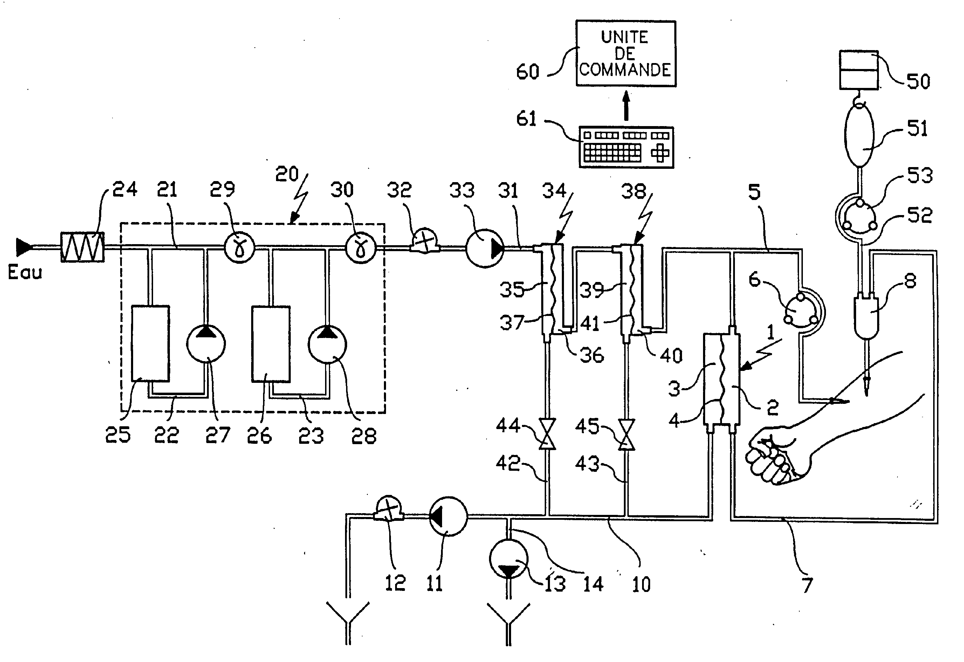

D'autres caractéristiques et avantages de l'invention apparaîtront mieux à la lecture de la description qui va suivre. On se reportera à l'unique figure annexée qui représente de façon schématique un appareil d'hémofiltration selon l'invention.

L'appareil d'hémofiltration représenté sur la figure comprend un échangeur 1 ayant deux compartiments 2, 3 séparés par une membrane semi-perméable 4. Un premier compartiment 2 a une entrée connectée à une canalisation 5 de prélèvement de sang (canalisation dite "artérielle") sur laquelle est disposée une pompe de circulation 6, et une sortie connectée à une canalisation 7 de restitution de sang (canalisation dite "veineuse") sur laquelle est interposé un piège à bulles 8. Le premier compartiment 2 de l'échangeur 1, la canalisation 5 de prélèvement de sang et la canalisation 7 de restitution de sang constituent le circuit pour la circulation extracorporelle de sang de l'appareil.

Le second compartiment 3 de l'échangeur 1 a une sortie connectée à une canalisation 10 d'évacuation de liquide usé (ultrafiltrat) dont l'extrémité libre est prévue pour être reliée à l'égout. Sur cette canalisation 10 sont disposés, dans le sens de circulation du liquide, une première pompe 11 et un débitmètre 12. Une deuxième pompe 13 est disposée sur une canalisation 14 d'extraction connectée à la canalisation 10 d'évacuation, en amont de la première pompe 11.

L'appareil d'hémofiltration selon l'invention comprend en outre un premier dispositif pour injecter une première solution dans le circuit de circulation extracorporelle de sang en amont de l'échangeur 1. Ce dispositif d'injection comprend un générateur 20 de liquide à usage médical apte à produire une solution de composition donnée par dilution d'au moins une solution concentrée. Le générateur 20 représenté est constitué par une canalisation principale 21 à laquelle sont connectées en série deux canalisations de dérivation secondaires 22, 23. L'extrémité amont de là canalisation principale 21 est prévue pour être raccordée à une source d'eau courante qui peut être portée à la température souhaitée au moyen d'un organe de chauffage 24. Chaque canalisation secondaire 22 (23) comprend des moyens de raccord pour le montage d'une cartouche 25 (26) contenant un sel sous forme de granulés. Une pompe 27 (28) est disposée sur chaque canalisation secondaire 22 (23) en aval de la cartouche 25 (26) correspondante pour y faire circuler le liquide provenant de la canalisation principale. Chaque pompe 27 (28) est pilotée en fonction de la comparaison entre 1) une valeur de consigne de conductivité pour le mélange de liquides se formant à la jonction de la ligne principale 21 et de l'extrémité aval de la ligne secondaire 22 (23) et 2) la valeur de la conductivité de ce mélange mesurée au moyen d'une sonde de conductivité 29 (30) disposée sur la canalisation principale 21 immédiatement en aval de la jonction entre la canalisation principale 21 et l'extrémité aval de la canalisation secondaire 22 (23).

Le générateur 20 de solution est relié au circuit de circulation extracorporel de sang au moyen d'une canalisation 31 d'injection ayant une extrémité connectée à la canalisation principale 21 du générateur 20 et son autre extrémité connectée à la canalisation 5 de prélèvement de sang, en aval de la pompe à sang 6. Sur cette canalisation 31 d'injection sont disposés, dans le sens de circulation du liquide, un débitmètre 32, une pompe de circulation 33, un premier filtre 34 ayant deux chambres 35, 36 séparées par une membrane d'ultrafiltration 37 et un second filtre 38 ayant deux chambres 39, 40 séparées par une membrane d'ultrafiltration 41. La chambre 35 (39) de chacun des deux filtres 34, 38 où est introduite la solution à filtrer a une sortie reliée à la canalisation 10 d'évacuation par une canalisation d'entretien 42 (43) sur laquelle est disposée un vanne 44 (45).

L'appareil d'hémofiltration selon l'invention comprend aussi un second dispositif pour perfuser une seconde solution dans le circuit de circulation extracorporelle de sang, en aval de l'échangeur 1. Ce dispositif de perfusion

**4**

comprend une balance 50 prévue pour peser une poche 51 de solution qui est connectée à la canalisation 7 de restitution de sang par une canalisation 52 de perfusion sur laquelle est disposée une pompe 53. La balance 50 est utilisée pour piloter la pompe 51 pour que le débit du liquide de perfusion soit égal à un débit de consigne.

Le système d'hémofiltration représenté sur la figure comprend également une unité de calcul et de commande 60. Cette unité est reliée à un clavier alphanumérique 61 au moyen duquel un opérateur peut lui communiquer des instructions, telles que diverses valeurs de consigne. Par ailleurs, elle reçoit des information émises par les organes de mesure du système, tels que les débitmètres 12, 32, les sondes de conductivité 29, 30 et la balance 50. Elle pilote, en fonction des instructions reçues et de modes d'opération et d'algorithmes programmés, les organes moteurs du système, tels que les pompes 6, 11, 13, 27, 28, 33, 53 et les vannes 44, 45.

Dans le mode de réalisation de l'invention représenté sur la figure,

- la cartouche 25 ne contient que du bicarbonate de sodium;
- la cartouche 26 ne contient que du chlorure de sodium; et
- la poche 51 de liquide de perfusion contient une solution de chlorure de calcium, de magnésium et de potassium. Eventuellement, la poche 51 contient aussi du sodium.

L'appareil d'hémofiltration qui vient d'être décrit fonctionne de la façon suivante.

Un opérateur communique à l'unité de commande 60, par l'intermédiaire du clavier 61, des valeurs de consignes correspondant aux divers paramètres du traitement (prescription), à savoir, le débit du sang $Q_B$, le débit de la première solution Qpre, la perte de poids totale WL (quantité d'eau plasmatique à retirer du patient par ultrafiltration), la durée totale T de la séance, la concentration en bicarbonate $[HCO3^-]_{pre}$ de la première solution, la concentration en sodium $[Na^+]_{pre}$ de la première solution, la concentration en potassium $[K^+]_{post}$ de la deuxième solution, et la concentration en potassium $[K^+]_{des}$ vers laquelle la concentration du milieu intérieur du patient doit tendre.

Conformément à l'invention, l'unité 60 de commande et de calcul calcule alors le débit $Q_{post}$ de la deuxième solution qui est nécessaire pour compenser les pertes convectives qui se produisent dans l'échangeur (en particulier les pertes de potassium) et, éventuellement, pour corriger la concentration en potassium du milieu intérieur du patient et la faire tendre vers la concentration souhaitée $[K^+]_{des}$. La formule utilisée pour effectuer ce calcul, qui est stockée dans une mémoire de l'unité de commande 60, est la suivante:

$$Qpost = \frac{[K^+]des \times QB \times Qpre}{[K^+]post \times QB + [K^+]post \times Qpre - [K^+]des \times QB}$$

Conformément à l'invention, l'unité 60 de commande et de calcul est programmée pour recalculer le débit Qpost de la deuxième solution chaque fois que l'un des paramètres du traitement est modifié, en particulier le débit du sang $Q_B$ et le débit de la première solution $Q_{pre}$.

Après qu'une cartouche 25 de bicarbonate de sodium et qu'une cartouche 26 de chlorure de sodium ont été connectées aux canalisations 22, 23 correspondantes du générateur 20 de solution, la canalisation principale 21 est raccordée à une source d'eau courante et les pompes 27, 28, 33, 11 sont mises en fonctionnement. Les vannes 44, 45 disposées sur les canalisations d'entretien 42, 43 sont ouvertes, de sorte que, dans une phase initiale de fonctionnement de l'appareil, le liquide produit par le générateur de solution 20 est envoyé à l'égout. Les pompes 27 et 28 sont pilotées par l'unité de commande 60 de façon que la concentration en bicarbonate et la concentration en sodium de la première solution soient égales aux valeurs de consigne correspondantes $[HCO3^-]_{pre}$ et $[Na^+]_{pre}$. La pompe 33 d'injection de première solution est pilotée par l'unité de commande 60 de façon que son débit soit égal au débit de consigne $Q_{pre}$ (300 ml/min., par exemple) et le débit de la pompe 11 de circulation de liquide usé est réglé par l'unité de commande 60 de façon que les débits mesurés par les débitmètres 32, 12 soient égaux. Lorsque la première solution a la concentration souhaitée en sodium et en bicarbonate, la vanne 44 est fermée tandis que la vanne 45 est laissée ouverte pendant quelques instants jusqu'à ce que la première chambre du deuxième filtre 38 ait été rincée et remplie de solution, ainsi que la deuxième canalisation 43 d'entretien. Après la fermeture de la vanne 45, la première solution est filtrée une deuxième fois et cette solution, qui est désormais injectable, est utilisée pour rincer et remplir le circuit de circulation extracorporelle de sang (canalisation de prélèvement 5, premier compartiment 2 de l'échangeur 1, canalisation de restitution 7).

Lorsque l'amorçage (c'est-à-dire le rinçage et le remplissage) du circuit d'injection de la première solution et l'amorçage du circuit sang sont achevés, le circuit sang est connecté au patient et le traitement proprement dit peut commencer : les pompes 27, 28 du générateur 20 de solution, ainsi que la pompe 33 d'injection de solution et la pompe 11 de circulation du liquide usé continuent de fonctionner comme pendant l'amorçage du circuit, tandis que la pompe à sang 6, la pompe d'extraction 13 et la pompe de perfusion 53 sont mises en fonctionnement. La pompe à sang 6 est réglée au débit de consigne $Q_B$, (300 ml/mn, par exemple) et la pompe d'extraction 13 est réglée à un débit calculé par l'unité de commande 60 et égal à la somme du débit de perte de poids WL/T et du débit $Q_{post}$ de perfusion de la seconde solution. Conformément à l'invention, la pompe de perfusion 53 est réglée au débit $Q_{post}$ calculé par l'unité de commande comme mentionné ci dessus.

Exemple:

Les paramètres d'un traitement par hémofiltration administré par l'appareil selon l'invention sont définis comme suit:

- débit sang $Q_B$ = 300 ml/min.

- débit de la première solution = 300 ml/min.

- composition de la première solution:
Si l'objectif à atteindre, en terme de concentration du sodium $[Na^+]_{des}$ dans le milieu intérieur est de 141 millimoles, alors la concentration en sodium de la première solution est réglée à $[Na^+]_{pre}$ = 147 millimoles/litre.
Si l'objectif à atteindre, en terme de concentration de bicarbonate $[HCO3^-]_{des}$ dans le milieu intérieur est de 33,5 millimoles, alors la concentration en bicarbonate de la première solution est réglée à $[HCO3^-]_{pre}$ = 35 millimoles/litre.

- composition de la deuxième solution: on choisit un liquide de perfusion isotonique au sang et dans lequel les proportions relatives des ions soient égales aux proportions relatives souhaitées de ces mêmes ions dans le milieu intérieur du patient. Soit, par exemple, le liquide de perfusion de composition suivante:

    $[K^+]$ = 60 millimoles/litre
    $[Mg^{++}]$ = 15 millimoles/litre
    $[Ca^{++}]$ = 45 millimoles/litre
    $[Cl^-]$ = 180 millimoles/litre

- débit de la deuxième solution: si l'objectif à atteindre, en terme de concentration de potassium $[K^+]_{des}$ dans le milieu intérieur est de 2,5 millimoles, alors le débit de la pompe de perfusion de la deuxième solution doit être réglé à 6,4 ml/min., par application de l'équation (1).

Dans l'exemple qui précède, l'objectif à atteindre a été défini en terme de concentration de potassium $[K^+]_{des}$ dans le milieu intérieur. Il va de soi que lorsque le calcium ou le magnésium est considéré comme la substance critique pour tel ou tel patient, la pompe de perfusion 53 sera régulée en fonction d'un objectif défini en terme de concentration souhaitée de calcium $[Ca^{++}]_{des}$ ou de magnésium $[Mg^{++}]_{des}$ dans le milieu intérieur du patient.

Par ailleurs, dans le mode de réalisation de l'invention qui a été décrit plus haut, il n'est possible de faire tendre le milieu intérieur du patient vers une concentration déterminée que pour l'une des substances ioniques contenues dans la seconde solution. Si l'on souhaite viser plusieurs objectifs en terme de concentration des substances ioniques contenues dans la seconde solution, il suffit d'utiliser plusieurs poches de liquide de perfusion contenant chacune une seule substance ionique à doser et de prévoir autant de moyens de perfusion (balance et pompe). On peut aussi n'utiliser qu'une seule balance, à laquelle les différentes poches sont suspendues, et qu'une seule pompe associée à des moyens de distribution pour relier tour à tour, selon une séquence temporelle déterminée, chaque poche et le circuit de circulation extracorporelle de sang.

L'invention qui vient d'être décrite est susceptible de variantes, notamment en ce qui concerne la composition de la première solution et de la deuxième solution, et la préparation de la première solution injectable.

Pourvu que les principales substances ioniques du sang soient présentes dans l'une et/ou l'autre solution, la composition ionique de la première solution et de la deuxième solution peut être choisie très librement. De préférence, pour les raisons qui ont été exposées plus haut, les substances ioniques qui forment des précipités carbonatés quand le pH de la solution où ils sont présents est supérieur à environ 7,5 sont séparées et n'entrent dans la composition que de l'une ou de l'autre des deux solutions.

C'est ainsi que la deuxième solution peut ne contenir que du bicarbonate de sodium et la première solution contenir les principales substances ioniques du sang à l'exclusion du bicarbonate (savoir: chlore, sodium, magnésium, calcium, potassium). La première solution peut alors être préparée à partir d'une seule solution concentrée liquide, et le générateur de solution de l'appareil comprend alors des moyens de dilution pour diluer une seule solution concentrée (une pompe, régulée au moyen d'une sonde de conductivité, pour injecter la solution concentrée dans une canalisation principale reliée à une source d'eau courante). Avec un tel agencement, il est possible de faire tendre le milieu intérieur du patient vers deux valeurs de prescription, une concentration en sodium $[Na^+]_{des}$ et une concentration en bicarbonate $[HCO3^-]_{des}$.

La première solution peut aussi être préparée à partir de deux solutions concentrées liquides contenant les mêmes substances ioniques dans les mêmes concentrations, à l'exception du potassium dont la concentration dans l'une et l'autre solutions concentrées est différente. Le générateur de solution de l'appareil d'hémofiltration est alors similaire à celui qui a été décrit plus haut en relation à la figure annexée. En pilotant judicieusement les deux pompes utilisées pour injecter les deux solutions concentrées dans la canalisation principale du générateur de solution, la concentration en potassium de la première solution peut être ajustée selon les besoins de chaque patient particulier. Avec un tel agencement, il est possible de faire tendre le milieu intérieur du patient vers trois valeurs de prescription, une concentration en sodium $[Na^+]_{des}$, une concentration en potassium $[K^+]_{des}$ (au moyen de la première solution) et une concentration en bicarbonate $[HCO3^-]_{des}$ (au moyen de la deuxième solution).

Pour le cas où la substance A dont on souhaite faire tendre la concentration dans le milieu intérieur du patient vers une concentration souhaitée $[A]_{des}$ est présente non plus seulement dans la deuxième solution mais à la fois dans la première solution et dans la deuxième solution (par exemple, le potassium), il faut en tenir compte dans le calcul du débit de perfusion $Q_{post}$ de la deuxième solution. La formule à partir de laquelle l'unité de commande et de calcul 60 calcule alors le débit de perfusion Qpost de la deuxième solution est la suivante:

$$[A]_{des} \times (Q_{pre} + Q_{post}) \times QB/(QB + Q_{pre}) = ([A]_{pre} \times Q_{pre}) + ([A]_{post} \times Q_{post}) \quad (2)$$

Dans l'exemple de réalisation qui a été décrit plus haut, la première solution préparée par le générateur de solution 20 est rendue injectable par filtration au travers des deux filtres 34 et 38. Cette première solution pourrait naturellement être rendue injectable par d'autres moyens, en particulier par une installation de stérilisation à la chaleur telle que décrite dans le document FR 99 04207.

## Revendications

1. Appareil d'hémofiltration destiné à coopérer avec un hémofiltre (1) ayant un premier et un second compartiments (2, 3) séparés par une membrane semi-perméable (4), le premier compartiment (2) ayant une entrée connectable à une canalisation (5) de prélèvement de sang et une sortie connectable à une canalisation (7) de restitution de sang, et le second compartiment (3) ayant une sortie connectable à une canalisation (10) d'évacuation de liquide usé, l'appareil d'hémofiltration comportant:

   des moyens (20, 24, 34, 38) pour préparer une première solution injectable à partir d'au moins une solution concentrée;
   des moyens (33) pour injecter, à un débit d'injection Qpre, la première solution dans la canalisation (5) de prélèvement de sang;
   des moyens (50, 51, 52, 53) pour perfuser à un patient une deuxième solution contenant au moins une substance ionique A ayant une concentration déterminée [A]post différente de la concentration [A]pre de cette substance A dans la première solution;

   **caractérisé en ce qu'**il comporte :

   des moyens (60) pour déterminer un débit de perfusion Qpost de la deuxième solution pour que la concentration de la substance A dans le milieu intérieur du patient tende vers une concentration souhaitée [A]des, en fonction de la concentration [A]post de la substance A dans la deuxième solution, de la concentration souhaitée [A]des, du débit d'injection Qpre de la première solution, de la concentration [A]pre de la substance A dans la première solution et du débit du sang QB.

2. Appareil selon la revendication 1, **caractérisé en ce que** la substance A est absente de la première solution et **en ce que** les moyens de détermination du débit de perfusion $Q_{post}$ comprennent des moyens (60) de calcul pour calculer le débit de perfusion $Q_{post}$ selon la formule :

$$Q_{post} = \frac{[A]_{des} \times Q_B \times Q_{pre}}{[A]_{post} \times Q_B + [A]_{post} \times Q_{pre} - [A]_{des} \times Q_B}$$

**3.** Appareil selon une des revendications 1 et 2, **caractérisé en ce qu'**il comprend en outre des moyens de commande (60) pour piloter les moyens de perfusion (50, 51, 52, 53) de façon que le débit de la solution de perfusion soit sensiblement égal au débit déterminé $Q_{post}$.

**4.** Appareil selon une des revendications 1 à 3, **caractérisé en ce que** les moyens (20) pour préparer la première solution injectable comprennent:

des premiers moyens (27) de dilution pour diluer une première solution concentrée de chlorure de sodium, des seconds moyens (28) de dilution pour diluer une deuxième solution concentrée de bicarbonate de sodium, - des moyens (29, 30, 60) pour régler les premiers (27) et les seconds (28) moyens de dilution pour que la première solution ait une concentration en sodium déterminée et une concentration en bicarbonate déterminée.

**5.** Appareil selon la revendication 4, **caractérisé en ce que** les moyens (20) pour préparer la première solution injectable comprennent des moyens (26, 28) pour produire la première solution concentrée à partir de chlorure de sodium sous forme de poudre ou de granulés, et des moyens (25, 27) pour produire la deuxième solution concentrée à partir de bicarbonate de sodium sous forme de poudre ou de granulés.

**6.** Appareil selon une des revendications 4 et 5, **caractérisé en ce que** la deuxième solution comprend du calcium, du potassium et du magnésium et **en ce que** la substance A est le calcium, le potassium ou le magnésium.

**7.** Appareil selon une des revendications 1 à 3, **caractérisé en ce que** les moyens pour préparer la première solution injectable comprennent:

des moyens de dilution pour diluer une solution concentrée de chlorures de sodium, de calcium, de potassium et de magnésium ; des moyens pour régler les moyens de dilution pour que la première solution ait une concentration en sodium déterminée.

**8.** Appareil selon une des revendications 1 à 3, **caractérisé en ce que** les moyens pour préparer la première solution injectable comprennent:

des premiers moyens de dilution pour diluer une première solution concentrée de chlorures de sodium, de calcium, de potassium et de magnésium, dans laquelle le potassium a une première concentration; des seconds moyens de dilution pour diluer une deuxième solution concentrée de chlorures de sodium, de calcium, de potassium et de magnésium, dans laquelle le potassium a une deuxième concentration, - des moyens pour régler les premiers et les seconds moyens de dilution pour que le potassium ait une troisième concentration déterminée dans la première solution.

**9.** Appareil selon la revendication 7 ou 8, **caractérisé en ce que** la deuxième solution comprend du bicarbonate de sodium et **en ce que** la substance A est le bicarbonate.

**10.** Appareil selon une des revendications 1 à 9, **caractérisé en ce que** l'une des première et deuxième solutions contient du bicarbonate et ne contient pas de magnésium ni de calcium, tandis que l'autre solution contient du magnésium et du calcium et ne contient pas de bicarbonate

**11.** Appareil selon une des revendications 1 à 10, **caractérisé en ce que** les moyens pour préparer la première solution injectable comprennent des moyens (34, 38) pour filtrer la première solution.

**12.** Appareil selon une des revendications 1 à 11, **caractérisé en ce que** les moyens de perfusion (50, 51, 52, 53) sont prévus pour être connectés à la canalisation (7) de restitution de sang.

**13.** Appareil selon une des revendications 1 à 12, **caractérisé en ce qu'**il comprend un hémofiltre (1) ayant un premier et un second compartiments (2, 3) séparés par une membrane semi-perméable (4), le premier compartiment (2) ayant une entrée connectée à une canalisation (5) de prélèvement de sang et une sortie connectée à une canalisation (7) de restitution de sang, et le second compartiment (3) ayant une sortie connectée à un canalisation (10) d'évacuation de liquide usé,

**Claims**

**1.** A hemofiltration device designed to cooperate with a hemofilter (1) having a first and a second compartment (2, 3) separated by a semipermeable membrane (4), the first compartment (2) having an inlet that can be connected to a channel (5) for blood withdrawal and an outlet that can be connected to a channel (7) for blood return, and the second compartment (3) having an outlet that can be connected to a channel (10) for draining waste liquid, the hemofiltration device comprising:

means (20, 24, 34, 38) for preparing a first injectable solution starting from at least one concentrated solution;
means (33) for injection at an injection rate Qpre the first solution into the channel (5) for blood withdrawal;
means (50, 51, 52, 53) for perfusing a patient with a second solution containing at least one ionic substance A having a given concentration [A]post differing from the concentration [A]pre of said substance A in the first solution;

**characterized in that** it comprises:

means (60) for determining a perfusion flow rate Qpost of the second solution, so that the concentration of substance A inside the patient tends towards a desired concentration [A]des, as a function of the concentration [A]post of substance A in the second solution, of the desired concentration [A]des, of the injection flow rate Qpre of the first solution, of the concentration [A]pre of substance A in the first solution and of blood flow rate QB.

**2.** The device according to claim 1, **characterized in that** substance A is absent from the first solution and **in that** the means for determining the perfusion flow rate $Q_{post}$ comprise means (60) for calculating the perfusion flow rate $Q_{post}$ according to formula:

$$Q_{post} = \frac{[A]_{des} \times Q_B \times Q_{pre}}{[A]_{post} \times Q_B + [A]_{post} \times Q_{pre} - [A]_{des} \times Q_B}$$

**3.** The device according to one of claims 1 and 2, **characterized in that** it further comprises control means (60) for controlling the perfusion means (50, 51, 52, 53) so that the perfusion solution flow rate is basically the same as the given flow rate $Q_{post}$.

**4.** The device according to one of claims 1 to 3, **characterized in that** the means (20) for preparing the first injectable solution comprise:

first means (27) for diluting a first concentrated solution of sodium chloride,
second means (28) for diluting a second concentrated solution of sodium bicarbonate,
means (29, 30, 60) for adjusting the first (27) and second (28) dilution means so that the first solution has a given sodium concentration and a given bicarbonate concentration.

**5.** The device according to claim 4, **characterized in that** the means (20) for preparing the first injectable solution comprise means (26, 28) for producing the first concentrated solution starting from sodium chloride as powder or granulate, and means (25, 27) for producing the second concentrated solution starting from sodium bicarbonate as powder or granulate.

**6.** The device according to one of the claims 4 and 5, **characterized in that** the second solution comprises calcium, potassium and magnesium, and **in that** substance A is calcium, potassium or magnesium.

**7.** The device according to one of the claims 1 to 3, **characterized in that** the means for preparing the first injectable solution comprise:

means for diluting a concentrated solution of sodium, calcium, potassium and magnesium chloride;
means for adjusting the dilution means so that the first solution has a given sodium concentration.

8.  The device according to one of the claims 1 to 3, **characterized in that** the means for preparing the first injectable solution comprise:

first means for diluting a first concentrated solution of sodium, calcium, potassium and magnesium chloride, in which potassium has a first concentration;
second means for diluting a second concentrated solution of sodium, calcium, potassium and magnesium chloride, in which potassium has a second concentration;
means for adjusting the first and second dilution means so that potassium has a third given concentration in the first solution.

9.  The device according to claim 7 or 8, **characterized in that** the second solution comprises sodium bicarbonate and **in that** substance A is bicarbonate.

10.  The device according to one of the claims 1 to 9, **characterized in that** one of the first and second solutions contains bicarbonate and contains neither magnesium nor calcium, whereas the other solution contains magnesium and calcium and contains no bicarbonate.

11.  The device according to one of the claims 1 to 10, **characterized in that** the means for preparing the first injectable solution comprise means (34, 38) for filtering the first solution.

12.  The device according to one of the claims 1 to 11, **characterized in that** the perfusion means (50, 51, 52, 53) are designed to be connected to the channel (7) for blood return.

13.  The device according to one of the claims 1 to 12, **characterized in that** it comprises a hemofilter (1) having a first and a second compartment (2, 3) separated by a semipermeable membrane (4), the first compartment (2) having an inlet connected to a channel (5) for blood withdrawal and an outlet connected to a channel (7) for blood return, and the second compartment (3) having an outlet connected to a channel (10) for draining waste liquid.

**Patentansprüche**

1.  Hämofiltrationsgerät zur Zusammenwirkung mit einem Hämofilter (1), der einen ersten und einen zweiten Abteil (2, 3) aufweist, die durch eine semipermeable Membran (4) voneinander getrennt sind, wobei der erste Abteil (2) einen mit einem Kanal (5) zur Blutentnahme verbindbaren Eingang und einen mit einem Kanal (7) zur Blutrückgabe verbindbaren Ausgang besitzt, und wobei der zweite Abteil (3) einen mit einem Kanal (10) zum Ablauf von gebrauchter Flüssigkeit verbindbaren Ausgang besitzt,
wobei das Hämofiltrationsgerät:

Mittel (20, 24, 34, 38) zur Vorbereitung einer ersten einspritzbaren Lösung aus mindestens einer konzentrierten Lösung;
Mittel (33) zum Einspritzen bei einem Einspritzrate Qpre der ersten Lösung in den Kanal (5) zur Blutentnahme;
Mittel (50, 51, 52, 53) zur Perfusion in einen Patienten einer zweiten Lösung enthaltend mindestens einen ionischen Stoff A mit einer bestimmten Konzentration [A]post, die verschieden ist von der Konzentration [A]pre von diesem Stoff A in der ersten Lösung, umfasst;

**dadurch gekennzeichnet, daß** es:

Mittel (60) zur Bestimmung einer Perfusionsrate Qpost der zweiten Lösung, so daß die Konzentration des Stoffs A in der Innenumgebung des Patienten zu einer gewünschten Konzentration [A]des neigt, abhängig von der Konzentration [A]post des Stoffs A in der zweiten Lösung, von der gewünschten Konzentration [A]des, vom Einspritzrate Qpre der ersten Lösung, von der Konzentration [A]pre des Stoffs A in der ersten Lösung sowie von der Blutrate QB, umfasst.

**2.** Gerät nach Anspruch 1, **dadurch gekennzeichnet, daß** der Stoff A in der ersten Lösung nicht enthalten ist und daß die Mittel zur Bestimmung der Perfusionsrate $Q_{post}$ Mittel (60) zur Berechnung der Perfusionsrate $Q_{post}$ nach der Formel:

$$Q_{post} = \frac{[A]_{des} \times Q_B \times Q_{pre}}{[A]_{post} \times Q_B + [A]_{post} \times Q_{pre} - [A]_{des} \times Q_B}$$

umfassen.

**3.** Gerät nach einem der Ansprüche 1 und 2, **dadurch gekennzeichnet, daß** es Steuermittel (60) zum Steuern der Perfusionsmittel (50, 51, 52, 53) weiter umfasst, so daß die Rate der Perfusionslösung wesentlich gleich wie die bestimmte Rate $Q_{post}$ ist.

**4.** Gerät nach einem der Ansprüche 1 bis 3, **dadurch gekennzeichnet, daß** die Mittel (20) zur Vorbereitung der ersten einspritzbaren Lösung:

erste Mittel (27) zur Verdünnung einer ersten konzentrierten Lösung von Natriumchlorid,
zweite Mittel (28) zur Verdünnung einer zweiten konzentrierten Lösung von Natriumbikarbonat,
Mittel (29, 30, 60) zur Einstellung der ersten (27) und der zweiten (28) Verdünnungsmittel, so daß die erste Lösung eine bestimmte Natriumkonzentration und eine bestimmte Bikarbonatkonzentration besitzt, umfassen.

**5.** Gerät nach Anspruch 4, **dadurch gekennzeichnet, daß** die Mittel (20) zur Vorbereitung der ersten einspritzbaren Lösung Mittel (26, 28) zur Herstellung der ersten konzentrierten Lösung aus Natriumchlorid als Pulver oder Granulat, und Mittel (25, 27) zur Herstellung der zweiten konzentrierten Lösung aus Natriumbikarbonat als Pulver oder Granulat umfassen.

**6.** Gerät nach einem der Ansprüche 4 und 5, **dadurch gekennzeichnet, daß** die zweite Lösung Kalzium, Kalium und Magnesium umfasst und daß der Stoff A Kalzium, Kalium oder Magnesium ist.

**7.** Gerät nach einem der Ansprüche 1 bis 3, **dadurch gekennzeichnet, daß** die Mittel zur Vorbereitung der ersten einspritzbaren Lösung:

Mittel zur Verdünnung einer konzentrierten Lösung von Natrium-, Kalzium-, Kalium- und Magnesiumchlorid;
Mittel zur Einstellung der Verdünnungsmittel, so daß die erste Lösung eine bestimmte Natriumkonzentration besitzt, umfassen.

**8.** Gerät nach einem der Ansprüche 1 bis 3, **dadurch gekennzeichnet, daß** die Mittel zur Vorbereitung der ersten einspritzbaren Lösung:

erste Mittel zur Verdünnung einer ersten konzentrierten Lösung von Natrium-, Kalzium-, Kalium- und Magnesiumchlorid, worin Kalium eine erste Konzentration besitzt;
zweite Mittel zur Verdünnung einer zweiten konzentrierten Lösung von Natrium-, Kalzium-, Kalium- und Magnesiumchlorid, worin Kalium eine zweite Konzentration besitzt;
Mittel zur Einstellung der ersten und zweiten Verdünnungsmittel, so daß Kalium eine bestimmte dritte Konzentration in der ersten Lösung besitzt, umfassen.

**9.** Gerät nach Anspruch 7 oder 8, **dadurch gekennzeichnet, daß** die zweite Lösung Natriumbikarbonat umfasst und daß der Stoff A Bikarbonat ist.

**10.** Gerät nach einem der Ansprüche 1 bis 9, **dadurch gekennzeichnet, daß** eine von der ersten und zweiten Lösungen Bikarbonat enthält und weder Magnesium noch Kalzium enthält, während die andere Magnesium und Kalzium enthält und kein Bikarbonat enthält.

**11.** Gerät nach einem der Ansprüche 1 bis 10, **dadurch gekennzeichnet, daß** die Mittel zur Vorbereitung der ersten einspritzbaren Lösung Mittel (34, 38) zur Filtration der ersten Lösung umfassen.

**12.** Gerät nach einem der Ansprüche 1 bis 11, **dadurch gekennzeichnet, daß** die Perfusionsmittel (50, 51, 52, 53) vorgesehen sind, um mit dem Kanal (7) zur Blutrückgabe verbunden zu werden.

**13.** Gerät nach einem der Ansprüche 1 bis 12, **dadurch gekennzeichnet, daß** es einen Hämofilter (1) umfasst, mit einem ersten und einem zweiten Abteil (2, 3), die durch eine semipermeable Membran (4) voneinander getrennt sind, wobei der erste Abteil (2) einen mit einem Kanal (5) zur Blutentnahme verbundenen Eingang und einen mit einem Kanal (7) zur Blutrückgabe verbundenen Ausgang besitzt, und wobei der zweite Abteil (3) einen mit einem Kanal (10) zum Ablauf von gebrauchter Flüssigkeit verbundenen Ausgang besitzt.

UNITE
DE
COMMANDE

Eau

EP 1 097 724 B1

## RÉFÉRENCES CITÉES DANS LA DESCRIPTION

**Documents brevets cités dans la description**

- EP 0898975 A
- EP 0898976 A
- EP 0622087 A
- DE 19654746
- EP 622087 A
- FR 9904207

**Littérature non-brevet citée dans la description**

- **E. QUELLHORST ; U. HILDEBRAND ; A. SOLF.** *Contrib. Nephrol. Basel, Karger,* 1995, vol. 113, 110-119